# EUROPÄISCHE PATENTSCHRIFT

(11) **EP 0 345 571 B1**
(45) Veröffentlichungstag und Bekanntmachung des Hinweises auf die Patenterteilung: **20.01.1993**
(21) Anmeldenummer: 89109580.4
(22) Anmeldetag: 27.05.1989
(51) Int. Cl.: A61K 7/00, A61K 7/48, A61K 7/06

(54) **Pflanzliche Wirkstoffzubereitung zur Verwendung in Kosmetika**
Plant extract composition for cosmetical use
Composition à base d'extrait de plante pour l'utilisation en cosmétique

(30) Priorität: 09.06.1988 DE 3819594; 19.07.1988 DE 3824350
(43) Veröffentlichungstag der Anmeldung: 13.12.1989
(73) Patentinhaber: Singh-Verma, Shyam Bir, Dr., D-50169 Kerpen (DE)
(72) Erfinder: Singh-Verma, Shyam, Dr., D-5014 Kerpen 4 (DE); Jordan, Josef, D-5014 Kerpen 4 (DE)
(74) Vertreter: Naumann, Ulrich, Dr.-Ing.

(56) Entgegenhaltungen:
- EP-A- 0 294 808
- FR-A- 2 558 727
- ECONOMIC BOTANY, Band 14, 1960, Seiten 119-128; J.F. MORTON: "The emblic (Phyllanthus emblica L.)"

## Beschreibung

Die Erfindung betrifft eine kosmetische Verwendung einer Wirkstoffzubereitung zur Hautpflege.

Gerade in den letzten Jahren hat die wirkstoffhaltige Kosmetik (Wirkstoff-Kosmetik) ein zunehmendes Interesse erfahren, um auf die unterschiedlichen Hauttypen und/oder Hautzustände gezielt abgestimmte Pflegemittel schaffen zu können. Es werden hierzu Substanzen gesucht, die neben der gewünschten Wirksamkeit auch gesundheitlich unbedenklich sind, insbesondere bei der gesunden Haut ohne Nebenwirkungen auf Dauer angewendet werden können. Wirkstoffe pflanzlicher Herkunft spielen dabei eine bedeutende Rolle, wobei insbesondere der Rückgriff auf Arznei-Pflanzen den Vorteil bietet, daß ihre gesundheitliche Unbedenklichkeit zum Teil über Jahrhunderte empirisch und auch wissenschaftlich belegt ist.

Es ist aus der Veröffentlichung "Economic Botany", 14/1960, Seite 119-128 bekannt, Fruchtkerne der Amlapflanze (Phyllanthus emblica Linné) zu rösten, zu pulverisieren und mit einem Öl zu einer Hautsalbe zu verreiben. Eine derartige Hautsalbe kann wegen des hohen Feststoffanteils weder als kosmetisches Mittel noch als Pflegemittel für die gesunde Haut eingesetzt werden. Der Röstvorgang zerstört wesentliche für ein kosmetisches Mittel zur Hautpflege bedeutsame Inhaltsstoffe.

Aus der in Rede stehenden Veröffentlichung ist es des weiteren bekannt, Extrakte der Amlapflanze als Bräunungsmittel, Mundwasser, Haarwuchsmittel und Mittel gegen Augenentzündung zu verwenden.

Der Erfindung liegt nun die Aufgabe zugrunde, eine kosmetische Verwendung einer Wirkstoffzubereitung zu finden, die zum einen die Anforderung an die Verwendbarkeit und auch die Wirksamkeit und zum anderen die Anforderung an die gesundheitliche Unbedenklichkeit für ein zum täglichen Gebrauch bestimmtes Mittel zur Hautpflege erfüllt.

Diese Aufgabe wird gemäß der Erfindung gelöst durch die Merkmale des Patentanspruches 1, genauer gesagt durch die kosmetische Verwendung zur Hautpflege von Extrakten der Samenkerne und/oder Pflanzenbestandteile, insbesondere der frischen und/oder getrockneten Früchte und/oder Blätter und/oder Rinde der Amlapflanze (Phyllanthus emblica Linné) oder der Amlapflanze und teilweise der Chebulapflanze (Terminalie chebula Ritz). Hierbei hat es sich überraschend gezeigt, daß die Inhaltsstoffe der genannten Bestandteile der Amlapflanze und ggf. der Chebulapflanze in ihrer Zusammensetzung bei dem Einsatz als Wirkstoff in Haut- und Körperpflege-Präparaten besondere kosmetische und pflegende Eigenschaften bewirken, wie sie bisher noch nicht bekannt waren. Wichtig ist hierbei, daß die Inhaltsstoffe so als Wirkstoff zur Verfügung stehen, daß auch eine sofortige Einwirkung auf die Hautoberfläche gegeben ist. Hierzu ist es wichtig, die Inhaltsstoffe möglichst schonend und vollständig aus den Pflanzenbestandteilen der Amlapflanze bzw. Chebulapflanze zu extrahieren. Das anzuwendende Extraktionsverfahren richtet sich zum Teil auch nach der Art des Pflegemittels, für das der betreffende Extrakt verwendet werden soll. Die Inhaltsstoffe der Amlapflanze haben eine adstringierende, entfettende und - bei längerer Anwendung - auch die Tätigkeit der Talgdrüsen positiv beeinflussende und deutlich regulierende Wirkung. Bei Gesichts- und Hautpflege-Präparaten für fette oder Problemhaut ergibt sich bei höheren Einsatzkonzentrationen eine eindeutige kosmetische Wirkung. Die Haut wird gestrafft und bekommt ein glattes und geschmeidiges Aussehen ohne Mitesser und sonstige Hautunreinheiten. Bei niedriger Dosierung ergibt sich für trockene und Mischhaut eine ausgezeichnete tonisierende und regenerierende Wirkung. Ethanolische Tinkturen, insbesondere ethanolische Tinkturen aus Früchten, sind für Hautpflegemittel bei fettender Haut von Bedeutung.

Zur Darstellung der vorliegenden Erfindung wird der Begriff "Tinktur" in einem erweiterten Sinne gebraucht. Er umfaßt zum einen die Tinktur im eigentlichen Sinne, d.h. den mit Hilfe eines Lösungsmittels gewonnenen und in diesem enthaltenen Auszug aus den genannten Pflanzenbestandteilen. Er umfaßt aber auch den sogenannten Extrakt, bei dem die Wirkstoffe aus den genannten Pflanzenbestandteilen durch Lösungsmittel, ggf. in Verbindung mit Druck und/oder Temperatur gewonnen werden. Das jeweils verwendete Lösungsmittel bestimmt in beiden Fällen die Wirkstoffzusammensetzung nach Art und Menge. Im folgenden wird daher der Begriff "Tinktur" ohne weitere Unterscheidung sowohl für die Tinktur im eigentlichen Sinne, als auch für den Extrakt gebraucht, da die Gewinnung der Wirkstoffe nicht Gegenstand der Erfindung ist.

Von besonderer Bedeutung ist hierbei die Tinktur aus Früchten, die sowohl in frischer Form als auch in getrockneter Form verarbeitet werden können. Frische Amlafrüchte sind reich an Oleoresin, Gummi, Pektin sowie 0,415 % gut stabilisierter Ascorbinsäure. In getrocknetem Zustand sind mindestens 13 verschiedende Polyphenole im ethanolischen Extrakt darstellbar, darunter Ethylgallat, Gallussäure (5 %), Trigallyglucose, Terchebin, Corilagin, Chebulin-, Chebulagin- und Chebulininsäure sowie Glucose und 4 bis 9 % Schleimsäure. Daneben sind auch eine Reihe von Aminosäuren feststellbar, wie Prolin, Leucin, Valin, Asperagin, Arginin, Lysin und Threonin.

In Ausgestaltung der Erfindung ist ferner vorgesehen, daß die Wirkstoffzubereitung durch Samenkerne gebildet wird. Das Samenkernöl, das durch ein Extraktionsverfahren gewonnen werden kann, ist ein sogenanntes halbtrockenes Öl und unterscheidet sich von fetten Ölen in erheblichem Maße dadurch, daß es ca. 78 % ungesättigte Fettsäuren, hauptsächlich Linol-, Linolen- und Ölsäuren, enthält. Der Rest besteht aus gesättigten Fettsäuren wie Capryl-, Laurin-, Palmetin- und Myristinsäure. Im Extraktionsverfahren gewonnenes Amla-Samenkernöl weist folgende Kennzahlen auf:

| | |
|---|---|
| Säurezahl | 34,0 |
| Verseifungszahl | 199 |
| Jodzahl | 123 |
| Peroxidzahl | 26 |
| Unverseifbares | 1,3 %. |

Gerade der hohe Anteil an ungesättigten Fettsäuren, insbesondere Linol- und Ölsäure, machen das Samenkernöl für den Einsatz in einem kosmetischen und/oder Körperpflegemittel besonders interessant.

Bei den Inhaltsstoffen der Tinkturen und des Samenkernöls der Amlapflanze handelt es sich um Stoffe mit hautpflegender bzw. kosmetischer Wirkung, die für sich bekannt sind. Gerade die Zusammensetzung und Verteilung in Extrakten aus Pflanzenbestandteilen der Amlapflanze stellt ein bisher nicht gekanntes, ausgewogenes und noch wirksameres "System" dar, das in dieser Weise synthetisch gar nicht darstellbar ist, zumal Spuren anderer, nur dem Pflanzenextrakt eigene Stoffe, auf synthetische Weise nicht zugefügt werden können, die gleichwohl im Zusammenwirken mit den genannten Inhaltsstoffen Wirkungseinfluß haben, so die verschiedenen Aminosäuren, Polysacchariden und eine Reihe von Mineralstoffen.

In weiterer Ausgestaltung der Erfindung ist vorgesehen, daß die Wirkstoffzubereitung durch eine ölige Zubereitung der Pflanzenbestandteile, insbesondere der frischen und/oder getrockneten Früchte, gebildet wird.

In weiterer Ausgestaltung der Erfindung ist vorgesehen, daß die Wirkstoffzubereitung durch den Fruchtsaft gebildet wird. Der Fruchtsaft kann hierbei sowohl als Frischsaft als auch in konservierter Form eingesetzt werden.

In Ausgestaltung der Erfindung ist vorgesehen, daß in einem kosmetischen, hautpflegenden Mittel der Anteil einer aus Tinktur bestehenden Wirkstoffzubereitung 0,05 bis 25 %, vorzugsweise 0,1 bis 10 %, beträgt.

Erfindungsgemäß ist ferner vorgesehen, daß in einem kosmetischen, hautpflegenden Mittel der Anteil einer aus Samenkernöl bestehenden Wirkstoffzubereitung 0,1 bis 90 %, vorzugsweise 0,5 bis 50 %, beträgt.

Erfindungsgemäß ist vorgesehen, daß in einem kosmetischen, hautpflegenden Mittel der Anteil einer öligen Wirkstoffzubereitung 0,1 bis 90 %, vorzugsweise 1,0 bis 50 %, beträgt.

Erfindungsgemäß beträgt in eiem kosmetischen, hautpflegenden Mittel der Anteil einer aus Fruchtsaft gebildeten Wirkstoffzubereitung 0,1 bis 98 %, vorzugsweise 1,0 bis 50 %.

In einer anderen Ausgestaltung der Erfindung ist vorgesehen, daß der Anteil an Samenkernöl der Amlapflanze in der Wirkstoffzubereitung zumindest teilweise durch Samenkernöl der Chebulapflanze (Terminalie chebula Ritz) substituiert wird. Die Fettsäurezusammensetzung des Chebula-Samenkernöls unterscheidet sich vom Amla-Samenkernöl insofern, als der Anteil an Linolsäure allein schon etwa 40 % beträgt. Außerdem enthält es etwa 34 % Ölsäure. Die gesättigten Fettsäuren, die ca. 23 % ausmachen, bestehen im wesentlichen aus Palmetinsäure (16 %) und Stearinsäure (6 %) sowie Spuren von Capryl-, Laurin- und Myristinsäuren. Auch beim Chebula-Samenkernöl ist gerade der höhere Anteil an ungesättigten Fettsäuren, insbesondere Linol- und Ölsäuren, für den Einsatz in Kosmetik- und Körperpflegemitteln besonderes interessant.

Zur Verdeutlichung des Anwendungsspektrums der erfindungsgemäßen Wirkstoffzubereitung werden nachstehend einige Formulierungen für kosmetische, hautpflegende Mittel als Beispiele angegeben:

| 1. After Shave Lotion | |
|---|---|
| Ethanol 96 Vol. % | 53,00 % |
| Wasser demineralisiert | 44,60 - 33,70 % vorzgw. 40,20% |
| Chebulakernöl | 0,10 - 2,00 % vorzgw. 0,50 % |
| Amla-Tinktur | 1,00 - 10,00 % vorzgw. 5,00 % |
| Parfümöl | 1,00 % |
| Allantoin | 0,25 % |
| Menthol | 0,05 % |

| 2. Pre Shave Lotion | |
|---|---|
| Ethanol 96 Vol. % | 75,76 % |
| Wasser demineralisiert | 10,04 - 19,04 vorzgw. 15,04 % |
| Amla-Tinktur | 1,00 - 10,00 % vorzgw. 5,00 % |
| Partialglycerid mittelkettiger Fettsäuren | 2,00 % |
| Dimethylsiloxan-Polymer | 1,00 % |
| Parfümöl | 1,00 % |
| Allantoin | 0,20 % |

| 3. Gesichtslotion (Gesichtstonic) | |
|---|---|
| Ethanol 96 Vol. % | 15,00 % |
| Polyolfettsäureester | 2,00 % |
| Amla-Tinktur | 1,00 - 10,00 % vorzgw. 5,00 % |
| Wasser demineralisiert | 70,00 - 79,00 % vorzgw. 75,00% |
| Glycerin | 3,00 % |

Für eine solche Lotion ließe sich alternativ auch der frische Amla-Saft einsetzen, wie aus nachfolgender Rezeptur hervorgeht:

| | |
|---|---|
| Ethanol 96 Vol. % | 15,00 % |
| Polyolfettsäureester | 2,00 % |
| Amla-Frischsaft (Frucht) | 2,00 - 50,00 % vorzgw. 25,00% |
| Wasser demineralisiert | 30,00 - 78,00 % vorzgw. 55,00% |
| Glycerin | 3,00 % |

In der Wirkung sind beide Gesichtslotionen vergleichbar. Auf Parfümöl wurde in dieser Rezeptur verzichtet, da Amla-Tinkturen selbst einen angenehmen aromatischen Geruch aufweisen.

| 4. Shampoo gegen fettendes Haar | |
|---|---|
| Mischung von milden, waschaktiven Substanzen | 40,00 % |
| Amla-Tinktur | 3,00 - 15,00 % vorzgw. 10,00% |
| Decamethylcyclopentasiloxan | 1,00 % |
| Konservierungsmittel-Lösung | 1,00 % |
| Verdicker | 2,00 % |
| Parfümöl | 0,50 % |
| Wasser demineralisiert | 40,50 - 52,50 % vorzgw. 45,50% |

| 5. Haarkur intensiv gegen fettendes Haar (Emulsionsform) | |
|---|---|
| Cetylstearylalkohol | 0,50 % |
| Gemisch Cetylstearylalkohol und nichtionogener Emulgator | 3,00 % |
| Ölsäuredecylester | 3,00 % |
| Amla-Tinktur | 5,00 % |
| Glyzerin 86 % | 3,00 % |
| Citronensäure | 0,30 % |
| Wasser demineralisiert | 85,00 % |
| Parfümöl | 0,20 % |

Die vorstehend angegebenen Formulierungen verwenden Tinktur und auch Frischsaft als Wirkstoffzubereitung.

Wegen ihrer adstringierenden, entfettenden und die Aktiviät der Talgdrüsen regulierenden Eigenschaften zeigen Amla-Frucht-Tinkturen und Frischsaft bei entsprechend höheren Einsatzkonzentraten (5 - 25 %) in Präparaten für ölige bzw. fettende Haut und sogenannte Problemhaut eindeutige kosmetische Wirkung. Die Haut wird gestrafft und bekommt ein glattes und geschmeidiges Aussehen ohne Mitesser und sonstige Hautunreinheiten.

Hingegen haben diese Tinturen in niedriger Dosierung zwischen 1 bis 5 % sowie die öligen Zubereitungen aus getrockneten Früchten bei trockener und Mischhaut eine exzellente tonisierende und regenerierende Wirkung.

Die nachstehend angegebenen Formulierungen verwenden Samenkernöl bzw. ölige Zubereitungen aus getrockneten Früchten als Wirkstoffzubereitung.

| 6. Shampoo gegen trockenes Haar | |
|---|---|
| Mischung aus milden, waschaktiven Substanzen | 33,00 % |
| Chebulakernöl | 0,20 - 2,00 % vorzgw. 0,50 % |
| Polyolfettsäureester | 3,00 - 5,00 % vorzgw. 4,00 % |
| Parfümöl | 0,50 % |
| Konservierungsmittel-Lösung | 1,00 % |
| Decamethylcyclopentasiloxan | 1,00 % |
| Amla-Tinktur | 1,00 - 10,00 % vorzgw. 5,00 % |
| Wasser demineralisiert | 47,50 - 60,30 % vorzgw. 55,00% |

| 7. Hautfunktionsöl | |
|---|---|
| Neutralöl | 5,00 - 65,00 % vorzgw. 45,00% |
| ölige Zubereitung aus Amla-Trockenfrucht | 5,00 - 50,00 % vorzgw. 20,00% |
| Isopropylpalmitat | 15,00 % |
| 2-Octyldodecanol | 9,00 % |
| Chebula Samenkernöl | 5,00 - 20,00 % vorzgw. 10,00% |
| Rosmarinöl | 0,35 % |
| Campfer | 0,10 % |
| Parfümöl | 0,50 % |
| BHT | 0,05 % |

| 8. Massageöl - Hautfunktionsöl | |
|---|---|
| Amla-Samenkernöl | 68,45 - 98,45 % vorzgw. 78,45% |
| ölige Zubereitung aus Amla-Trockenfrucht | 0,00 - 30,00 % vorzgw. 20,00% |
| Konservierungsmittel-Lösung | 1,00 % |
| BHT | 0,05 % |
| Parfümöl | 0,50 % |

| 9. Massageöl - Hautfunktionsöl | |
|---|---|
| Chebula-Samenkernöl | 68,45 - 98,45 % vorzgw. 78,45% |
| ölige Zubereitung aus Amla-Trockenfrucht | vorzugsweise 20,00 % |
| Konservierungsmittel-Lösung | 1,00 % |
| BHT | 0,05 % |
| Parfümöl | 0,50 % |

| 10. Haarwasser fettend | |
|---|---|
| Ethanol 96 Vol. % | 80,00 % |
| Chebulakernöl | 0,20 - 1,00 % vorzgw. 0,50 % |
| Amla-Tinktur | 1,00 - 10,00 % vorzgw. 5,00 % |
| Parfümöl | 0,50 % |
| Wasser demineralisiert | 8,50 - 18,30 % vorzgw. 14,00% |

| 11. Haarwasser nicht fettend | |
|---|---|
| Ethanol 96 Vol. % | 80,00 % |
| Amla-Tinktur | 1,00 - 5,00 % vorzgw. 2,00 % |
| Parfümöl | 0,50 % |
| Wasser demineralisiert | 14,50 - 18,50 % vorzgw. 17,50% |

Der Einsatz von halbtrockenen Samenkernölen von Amla und Chebula mit einem Anteil an ungesättigten Festtsäuren von 70 bis 80 % wirkt in jeder Emulsion als ausgezeichnetes Emollient. Die Anwendung solcher Präparate gewährleistet der Haut Spannkraft und Elastizität. Sie sieht glatt, geschmeidig und jugendlich aus.

Zusammenfassend kann gesagt werden, daß die genannten Wirkstoffe der Amla- und Chebula-Pflanze für den Einsatz in Kosmetik und Körperpflegemitteln ihre Berechtigung haben. Die Tinkturen aus frischen und getrockneten Früchten wegen ihrer adstringierenden, entfettenden aber auch tonisierenden Wirkung und die Samenkernöle wegen ihres hohen Gehalts an ungesäuerten Fettsäuren zur Behandlung trockener, schuppiger Haut und Haare.

Bei den Abteilen der vorstehend angegebenen Formulierungen handelt es sich jeweils um Gewichtsprozente. Die jeweilige Wirkstoffzubereitung kann hierbei einzeln oder aber auch in Mischung mit einer anderen Form der Wirkstoffzubereitung eingesetzt werden. Dies gilt grundsätzlich auch für die Verwendung von frischem Fruchtsaft, wobei jedoch für eine ausreichende Konservierung Sorge getragen werden muß, da frischer Fruchtsaft hohe Gehalte an verschiedenen Zuckern, Aminosäuren und Mineralien enthält, so daß ohne die Zugabe von entsprechenden Konservierungsmitteln ein unzulässiger Keimbefall zu befürchten ist. Die Mischung und/oder Substituierung der unterschiedlichen Wirkstoffzubereitungen bietet die Möglichkeit einer gezielten Einwirkung auf die Haut, da aufgrund der unterschiedlichen Herstellungsverfahren der einzelnen Wirkstoffzubereitungen auch unterschiedliche Wirkstoffbestandteile aus den Pflanzenbestandteilen gewonnen werden. Hinzu kommt noch, daß die Pflanzenbestandteile unter sich wieder abweichende Wirkstoffgehalte aufweisen, so daß durch die Auswahl der einzelnen Pflanzenbestandteile wie Blätter, Rinde, Früchte oder Samenkerne einzeln oder in Mischung ihrer Wirkstoffzubereitungen eine breite Variationsmöglichkeit besteht.

## Patentansprüche

1. Kosmetische Verwendung einer Wirkstoffzubereitung zur Hautpflege, dadurch **gekennzeichnet,** daß Extrakte der Samenkerne und/oder Pflanzenbestandteile, insbesondere der frischen und/oder getrockneten Früchte und/oder Blätter und/oder Rinde der Amlapflanze (Phyllantus emblica Linné) oder der Amlapflanze und teilweise der Chebulapflanze (Terminalie chebula Ritz) enthalten sind.

2. Kosmetische Verwendung einer Wirkstoffzubereitung nach Anspruch 1, gekennzeichnet durch ein aus den Samenkernen, vorzugsweise durch Extraktion, gewonnenes Öl.

3. Kosmetische Verwendung einer Wirkstoffzubereitung nach Anspruch 1 oder 2, gekennzeichnet durch eine ölige Zubereitung der Pflanzenbestandteile, insbesondere der frischen und/oder getrockneten Früchte.

4. Kosmetische Verwendung einer Wirkstoffzubereitung nach einem der Ansprüche 1 bis 3, gekennzeichnet durch die Verwendung des Fruchtsaftes.

5. Kosmetische Verwendung einer Wirkstoffzubereitung nach einem der Ansprüche 1 bis 4, dadurch gekennzeichnet, daß in einem kosmetischen, hautpflegenden Mittel der Anteil an der Wirkstoffzubereitung 0,05 bis 25 %, vorzugsweise 0,1 bis 10 %, beträgt.

6. Kosmetische Verwendung einer Wirkstoffzubereitung nach einem der Ansprüche 1 bis 5, dadurch gekennzeichnet, daß in einem kosmetischen, hautpflegenden Mittel der Anteil der Wirkstoffzubereitung 0,1 bis 90 %, vorzugsweise 0,5 bis 50 %, beträgt.

7. Kosmetische Verwendung einer Wirkstoffzubereitung nach einem der Ansprüche 1 bis 6, dadurch gekennzeichnet, daß in einem kosmetischen, hautpflegenden Mittel der Anteil einer aus einer öligen Zubereitung von Pflanzenbestandteil bestehenden Wirkstoffzubereitung 0,1 bis 90 %, vorzugsweise 1,0 bis 50 %, beträgt.

8. Kosmetische Verwendung einer Wirkstoffzubereitung nach einem der Ansprüche 1 bis 7, dadurch gekennzeichnet, daß in einem kosmetischen, hautpflegenden Mittel der Anteil an einer aus Fruchtsaft bestehenden Wirkstoffzubereitung 0,1 bis 98 %, vorzugsweise 1,0 bis 50 %, beträgt.

9. Kosmetische Verwendung einer Wirkstoffzubereitung nach einem der Ansprüche 1 bis 8, dadurch gekennzeichnet, daß in einem kosmetischen, hautpflegenden Mittel der Anteil an Öl der Samenkerne der Amlapflanze in der Wirkstoffzubereitung zumindest teilweise durch Öl aus den Samenkernen der Chebula-Pflanze (Terminalie chebula Ritz) substituiert ist.

## Claims

1. Cosmetic use of an extract composition for skin care, characterised in that it contains extracts of seed kernels and/or plant components, in particular the fresh and/or dried fruits and/or leaves and/or bark of the amla plant (Phyllantus emblica Linné) or the amla plant and, in part, the chebula plant (Terminalie chebula Ritz).

2. Cosmetic use of an extract composition according to Claim 1, characterised in that it contains an oil obtained, preferably by extraction, from the seed kernels.

3. Cosmetic use of an extract composition according to Claim 1 or 2, characterised in that it contains an oily extract from the plant components, in particular of the fresh and/or dried fruits.

4. Cosmetic use of an extract composition according to one of Claims 1 to 3, characterised by the inclusion of the juice of the fruit.

5. Cosmetic use of an extract composition according to one of Claims 1 to 4, characterised in that the quantity of the extract composition in a cosmetic skin care agent is 0.05 to 25 %, and preferably 0.1 to 10 %.

6. Cosmetic use of an extract composition according to one of Claims 1 to 5, characterised in that the quantity of extract composition in the cosmetic skin care agent is 0.1 to 90 %, preferably 0.5 to 50 %.

7. Cosmetic use of an extract composition according to one of Claims 1 to 6, characterised in that the quantity of an extract composition consisting of an oily extract from plant components, in a cosmetic skin care agent is 0.1 to 90 %, preferably 1.0 to 50 %.

8. Cosmetic use of an extract composition according to one of Claims 1 to 7, characterised in that the quantity of an extract composition consisting of fruit juice in a cosmetic skin care agent is 0.1 to 98 %, preferably 1.0 to 50 %.

9. Cosmetic use of an extract composition according to one of Claims 1 to 8, characterised in that, in a cosmetic skin care agent the quantity of oil of the seed kernels of the amla plant in the extract composition is substituted, at least part, by oil from the seed kernels of the chebula plant (Terminalie chebula Ritz).

## Revendications

1. Utilisation cosmétique d'une préparation active pour les soins de la peau, caractérisée en ce que la préparation contient des extraits de noyaux et/ou de parties de plantes, notamment des fruits frais et/ou séchés et/ou des feuilles et/ou des écorces de la plante amla (Phyllantus emblica Linné) ou de la plante amla et en partie de la plante chebula (Terminalie chebula Ritz).

2. Utilisation cosmétique d'une préparation active selon la revendication 1, caractérisée par une huile obtenue, de préférence par extraction, à partir des noyaux.

3. Utilisation cosmétique d'une préparation active selon la revendication 1 ou 2, caractérisée par une préparation huileuse des parties de plantes, notamment des fruits frais et/ou séchés.

4. Utilisation cosmétique d'une préparation active selon l'une des revendications 1 à 3, caractérisée par l'utilisation du jus de fruit.

5. Utilisation cosmétique d'une préparation active selon l'une des revendications 1 à 4, caractérisée en ce que dans un produit cosmétique pour les soins de la peau, le pourcentage de la préparation active est compris entre 0,05 et 25 % et de préférence entre 0,1 et 10 %.

6. Utilisation cosmétique d'une préparation active selon l'une des revendications 1 à 5, caractérisée en ce que dans un produit cosmétique pour les soins de la peau, le pourcentage de la préparation active est compris entre 0,1 et 90 % et de préférence entre 0,5 et 50 %.

7. Utilisation cosmétique d'une préparation active selon l'une des revendications 1 à 6, caractérisée en ce que dans un produit cosmétique pour les soins de la peau, le pourcentage d'une préparation active constituée par une préparation huileuse d'une partie de plante est comprise entre 0,1 et 90 % et de préférence entre 1,0 et 50 %.

8. Utilisation cosmétique d'une préparation active selon l'une des revendications 1 à 7, caractérisée en ce que dans un produit cosmétique pour les soins de la peau, le pourcentage d'une préparation active constituée à partir d'un jus de fruit est comprise entre 0,1 et 98 % et de préférence entre 1,0 et 50 %.

9. Utilisation cosmétique d'une préparation active selon l'une des revendications 1 à 8, caractérisée en ce que dans un produit cosmétique pour les soins de la peau, la partie formée par de l'huile des noyaux de la plante amla dans la préparation active est remplacée au moins en partie par de l'huile tirée des noyaux de la plante chebula (Terminalie chebula Ritz).
